# EUROPEAN PATENT APPLICATION

(11) **EP 3 563 770 A1**
(43) Date of publication of application: **06.11.2019**
(21) Application number: 18170322.4
(22) Date of filing: 02.05.2018
(51) Int. Cl.: A61B 8/08, A61B 8/12, A61B 8/00, A61B 5/00

(54) **INTRAVASCULAR NAVIGATION USING DATA-DRIVEN ORIENTATION MAPS**

(71) Applicant: Koninklijke Philips N.V., 5656 AE Eindhoven (NL)
(72) Inventor: WISSEL, Tobias, 5656 AE Eindhoven (NL); NICKISCH, Hannes, 5656 AE Eindhoven (NL); GRASS, Michael, 5656 AE Eindhoven (NL)
(74) Representative: de Haan, Poul Erik

(57) **Abstract**

A system SY for determining a position of an OCT or IVUS catheter CA in a vasculature VA. The system SY includes a map-providing unit MPU, a data-providing unit DPU, and a comparator unit COMP. The map-providing unit is configured to provide a reference map SOM including reference measurement data at each of a plurality of positions P_{1..k} of the OCT or IVUS catheter CA in the vasculature VA. The data-providing unit DPU is configured to provide actual measurement data from an actual position in the vasculature VA. The comparator unit COMP is configured to determine from the plurality of positions P_{1..k} of the OCT or IVUS catheter in the vasculature, a position P_{n ⊂ 1..k} that corresponds to the actual position in the vasculature, based on a comparison of the actual measurement data and the reference measurement data. The reference measurement data and the actual measurement data are either i) OCT data or ii) IVUS data.

## Description

### FIELD OF THE INVENTION

The invention relates to the field of intravascular imaging, and more particularly, to interventional cardiology.

### BACKGROUND OF THE INVENTION

A document [1] by Nair, A.; Kuban, B. D.; Tuzcu, E. M.; Schoenhagen, P.; Nissen, S. E. and Vince, D. G., entitled "Coronary plaque classification with intravascular ultrasound radiofrequency data analysis"; Circulation, 2002, 106, 2200-2206; discloses that spectral analysis of backscattered intravascular ultrasound, i.e. IVUS, data has potential for real-time in-vivo plaque classification.

A document [2] by João Silva Marques and Fausto J. Pinto, entitled "The vulnerable plaque: Current concepts and future perspectives on coronary morphology, composition and wall stress imaging"; Rev. Port. Cardiol. 33(2):101-110, 2014; reviews state-of-the-art cardiovascular imaging for identification of vulnerable plaque.

Another document US2013/0109958A1 relates to a system that orientates and displays intra-vascular ultrasound imaging data using an acquisition processor and an image data processor. The acquisition processor acquires, during navigation of an ultrasound catheter through a vessel, for multiple individual intra-vascular ultrasound images comprising an image sequence, data representing an individual image and orientation data associated with the individual image indicating orientation of the catheter and individual image with respect to a reference position external to patient anatomy. The image data processor aligns the multiple individual intra-vascular ultrasound images with respect to the external reference position by rotating individual images of the image sequence as necessary to have substantially the same orientation with respect to the external reference position in response to the orientation data.

### SUMMARY OF THE INVENTION

Intravascular ultrasound, i.e. IVUS, plays an increasing role in imaging in coronary as well as peripheral vessels. Application cases include the evaluation of the coronary plaque burden, stenosis characterization or imaging support in the context of stenting. Along similar lines, optical coherence tomography, i.e. OCT, has started to evolve into an imaging modality that may provide both similar and complementary information. Intravascular imaging is typically done before, during and after the intervention in order to plan, monitor and evaluate the result. This often involves examining several locations along the vessel that may be revisited several times in a workflow for pure imaging, sensing or other operations. Here, catheter navigation is typically supported by 2D angiography which involves the injection of contrast medium and radiation exposure for the patient and the interventional cardiologist.

In order to reduce the extent of required angiography, intravascular imaging is used to construct orientation maps along the vessel. These maps store pattern information for each visited location in the vessel and can indicate, via similarity measures between the stored and a currently recorded pattern, whether this or a close-by location has been visited before and where it is located in the vessel according to past recordings. The key idea for generating these patterns is that intravascular imaging penetrates the lumen and wall borders and classifies this acquired information into groups. These can include types of tissue but also simply re-appearing patterns of un-defined anatomical meaning. The mapping from imaging information into a discrete set of classes is achieved via (un)supervised statistical learning. The arrangement of classes across locations at the lumen/wall boundary yields identifying patterns similar to graffiti at the inner wall of a tunnel. Pattern information can also be paired with rough spatial features of the deformable vessel geometry.

Thereto a system for determining a position of an OCT or IVUS catheter in a vasculature, is provided. The system includes a map-providing unit, a data-providing unit, and a comparator unit. The map-providing unit is configured to provide a reference map including reference measurement data at each of a plurality of positions of the OCT or IVUS catheter in the vasculature. The data-providing unit is configured to provide actual measurement data from an actual position in the vasculature. The comparator unit is configured to determine from the plurality of positions of the OCT or IVUS catheter in the vasculature, a position that corresponds to the actual position in the vasculature, based on a comparison of the actual measurement data and the reference measurement data. The reference measurement data and the actual measurement data are either i) OCT data or ii) IVUS data.

In so doing the actual position can be determined with respect to the reference map. This improves workflow since it simplifies the re-visiting of previously-visited locations along the vessel. It may also improve the accuracy and/ or speed of reaching previously-visited locations along the vessel because, in contrast to known techniques which use a spatial encoder at the entry point of the catheter to determine said position, and which are subject to axial variations in path length and consequently a positioning error due to the malleable nature of the vasculature, in the invention the mapping to the previously-visited locations is exact. Another advantage is that since the data is IVUS or OCT data, the radiation dose to the patient may be reduced since there is less need for angiography.

In accordance with one aspect the actual measurement data and the reference measurement data are each classified with a feature set. The comparator unit determines the position that corresponds to the actual position in the vasculature by comparing the feature set of the actual measurement data with the feature set of the reference measurement data. The feature set may include, for example, an intensity distribution of the respective data in a radial direction and/ or a rotational direction with respect to an axis defined by a lumen of the vasculature, a representation of tissue type in a radial direction and/ or a rotational direction with respect to an axis defined by a lumen of the vasculature, a representation of plaque type in a radial direction and/ or a rotational direction with respect to an axis defined by a lumen of the vasculature, an average intensity of the respective data, an entropy of the respective data, a geometrical parameter of the vasculature such as an average diameter of the vasculature or a cross-sectional area of the vasculature or a plaque thickness or a vessel wall thickness. By classifying the data with such a feature set, a faster comparison may be made when re-visiting previously-visited positions in the vasculature.

In accordance with another aspect the actual measurement data and the reference measurement data are both measured in a radial direction with respect to the axis defined by the lumen of the vasculature and correspond to a region of the vasculature bordered by the lumen and a vessel wall. In this aspect the actual measurement data and the reference measurement data corresponding to the bordered region are both scaled in the radial direction to a common radial range such that the comparator unit determines the position that corresponds to the actual position in the vasculature by comparing the actual measurement data and the reference measurement data at corresponding radii within the common radial range. This scaling improves the reliability of the position determination because deformations in the vasculature, e.g. variations during the cardiac phase, may be compensated-for.

In accordance with another aspect the actual measurement data and reference measurement data each have an angular sampling interval rotationally around an axis defined by a lumen of the vasculature. In this aspect the angular sampling interval of the reference measurement data is less than the angular sampling interval of the actual measurement data. This allows for the use of "sparse" actual measurement data when re-visiting the vasculature and may provide a faster determination of the actual position with respect to the reference map.

In accordance with another aspect the actual measurement data and reference measurement data each have an axial sampling interval along an axis defined by the lumen of the vasculature. In this aspect the axial sampling interval of the reference measurement data is less than the axial sampling interval of the actual measurement data. This likewise allows for the use of "sparse" actual measurement data when re-visiting the vasculature and may provide a faster determination of the actual position with respect to the reference map.

In accordance with another aspect the actual measurement data and the reference measurement data have a penetration depth in a radial direction with respect to an axis defined by a lumen of the vasculature. In this aspect the penetration depth of the actual measurement data is less than the penetration depth of the reference measurement data. This likewise allows for the use of "sparse" actual measurement data when re-visiting the vasculature and may provide a faster determination of the actual position with respect to the reference map.

A corresponding method and computer program product are also provided in accordance with these and other related aspects.

### BRIEF DESCRIPTION OF THE FIGURES

Fig. 1 illustrates an exemplary system SY for determining a position of an OCT or IVUS catheter CA in a vasculature VA in accordance with some aspects of the invention.
Fig. 2 illustrates a flowchart corresponding to a method MET of determining a position of an OCT or IVUS catheter CA in a vasculature VA.
Fig. 3 illustrates pattern generation based on tissue typing using virtual histology.
Fig. 4: illustrates an exemplary IVUS imaging embodiment of the invention that uses pullback through a vessel while imaging the vessel.

### DETAILED DESCRIPTION OF THE INVENTION

In order to illustrate the principles of the present invention a system is described with particular reference to an IVUS catheter. The system is used to determine a position of the IVUS catheter in a vasculature. It is however to be appreciated that the system may alternatively be used to determine a position of an OCT catheter in the vasculature.

As mentioned above, IVUS plays an increasing role for the imaging in coronary as well as peripheral vessels. Application cases include the evaluation of the coronary plaque burden, stenosis characterization or imaging support in the context of stenting. Along similar lines, OCT has started to evolve into an imaging modality that may provide both similar and complementary information. Intravascular imaging is typically used before, during and after an intervention to plan, monitor and evaluate the result. This often involves examining several locations along the vessel that may be revisited several times in a workflow for imaging, sensing or other operations. Here, catheter navigation is typically supported by 2D angiography which involves the injection of contrast medium and radiation exposure for patient and interventional cardiologist.

While the clinician navigates the catheter through the vessel, several locations may be revisited more than just once. Images acquired during navigation usually have no adhoc associations with recordings acquired earlier in the intervention although they possibly originate from the very same location.

This may entail several drawbacks, including:
In order to navigate through the vessel and get an orientation where the catheter tip is currently located, repeated angiographic imaging is necessary. This involves the injection of contrast medium and radiation exposure for patient and interventional cardiologist.

Re-visiting specific sites-of-interest often requires time-consuming search which relies on angiographic and intravascular imaging as well as the interventional cardiologist's ability to coordinate, handle and match these different sources of information with his own memory.

Known systems lack a simple quantitative cross-check whether the correct site has actually been reached, and have no automatic information retrieval from earlier visits that could be compared to the current acquisition.

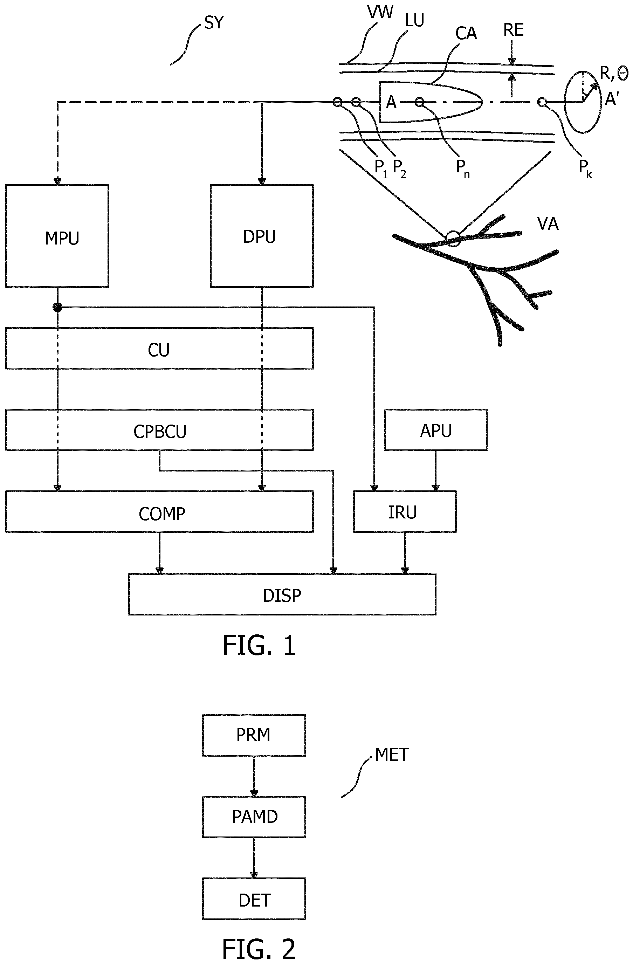

Thereto, Fig. 1 illustrates an exemplary system SY for determining a position of an OCT or IVUS catheter CA in a vasculature VA in accordance with some aspects of the invention. System SY includes map-providing unit MPU, data-providing unit DPU, and comparator unit COMP. Map-providing unit MPU is configured to provide a reference map including reference measurement data at each of a plurality of positions P_{1.k} of OCT or IVUS catheter CA in vasculature VA. Vasculature VA may be a portion of the vasculature of a human or animal body. The illustrated portion in Fig. 1 includes lumen LU that defines axis A - A', and along which axis positions P_{1.k} are defined. Lumen LU has a corresponding vascular wall VW, and region RE of vasculature VA is bordered by lumen LU and vessel wall VW. The reference measurement data of map-providing unit MPU is either i) OCT data or ii) IVUS data. This measurement data may correspond to detected OCT or IVUS signals that are measured rotationally, as indicated by angle θ, around axis A - A'. In the case of IVUS, the intensity of the data corresponds to the ultrasound reflectance of surrounding matter. In the case of OCT the data corresponds to interference between an optical irradiation beam and a reflected portion of the beam. The reference measurement data thus represents imaging data. By rotationally it is meant that the data is measured around a portion of angular direction θ. Said measurement data may for example be acquired from a fixed array of IVUS/ OCT detector elements, said elements being arranged around angular direction θ with respect to catheter CA, or from a scanning or rotating IVUS/ OCT detector wherein one or more detector elements, or a corresponding optical element, is rotated around angular direction θ with respect to catheter CA. Said measurement data is typically acquired during a so-called "pullback" procedure in which catheter CA, which has previously typically been inserted in vasculature VA with the assistance of a guidewire, is subsequently pulled-back along the vasculature whilst acquiring data. If desired, corresponding position data indicative of axis positions P_{1.k} may be determined in addition to the measurement data, for example using a spatial encoder at the entry point of the catheter, a well-defined pullback speed, or just be defined in terms of data order (i.e. "next", "previous" in a series of images) in case only the movement direction, but not the speed is known. The reference measurement data may be provided by catheter CA, i.e. the same catheter that provides actual measurement data to data-providing unit DPU, and as illustrated by the dashed line interconnecting catheter CA and map-providing unit MPU, or by another catheter. For example it may have been acquired from another catheter during an earlier examination procedure. In either case, map-providing unit MPU may be provided by a memory that stores said reference measurement data.

Data-providing unit DPU in Fig. 1 provides actual measurement data from an actual position in the vasculature VA. Thereto the actual measurement data may correspond to a live, or current position in vasculature VA, whereas the reference measurement data may correspond to historic positions in vasculature VA. The actual measurement data may be provided in the same manner as described above in relation to the reference measurement data. Preferably the actual measurement data is provided by catheter CA. Data-providing unit DPU may be provided by a memory that stores said actual measurement data.

In one particular implementation the actual measurement data corresponds to detected OCT or IVUS signals that are measured rotationally, as indicated in Fig. 1 by angle θ, around an axis A - A' defined by a lumen LU of the vasculature VA and the reference measurement data is simulated OCT or IVUS data indicative of OCT or IVUS signals measured rotationally around axis A - A' and which simulated data is derived from magnetic resonance imaging, i.e. MRI, data corresponding to the vasculature (VA). A technique for providing such simulated IVUS data from MRI image data is known for example from document: Imaging technologies for cardiac fiber and heart failure: a review, by Watson, S. R. et al., Heart Failure Reviews (2018) 23:273-289. This document describes the use of magnetic resonance diffusion tensor imaging, i.e. MR-DTI to characterize myocardial structures. Using MRI images in this manner shares the same benefit as the use of IVUS or OCT data; i.e. that of reduced radiation dose to a patient, as compared to a known technique in which CT images are used to determine a current position of an IVUS catheter in the vasculature.

Comparator unit COMP in Fig. 1 compares the actual measurement data and the reference measurement data, and based on said comparison, determines from the plurality of positions P_{1.k} of the OCT or IVUS catheter in the vasculature, a position P_{n⊂1..k} that corresponds to the actual position in the vasculature. Comparator unit COMP may for example compare the intensity of the IVUS/ OCT data in a radial, i.e. depth-wise direction at each position, in order to determine the actual position with respect to the reference map. A similarity metric such as a Euclidean distance between data values may be computed in order to determine a best-matched position for position P_{n ⊂ 1..k}. Other similarity metrics may be used in a similar manner. Thus, the reference measurement data provides a unique pattern that is indicative of a position in the reference map and allows the position of subsequently-acquired actual measurement data to be determined with respect to the reference map. In so doing, an accurate position may be determined. Optionally, in order to further improve the certainty of a match, data-providing unit DPU may provide additional measurement data from one or more positions having a known spatial relationship with the actual position in the vasculature (VA). Comparator COMP may use this additional data, in addition to the actual measurement data from the actual position in the vasculature VA, to determine from the plurality of positions (P_{1.k}) of the OCT or IVUS catheter in the vasculature, a position (P_{n ⊂ 1..k}) that corresponds to the actual position in the vasculature. Thus, for example, an IVUS cross-sectional IVUS image from a position axially on one side of the actual IVUS image, i.e. the actual measurement data, may be used to determine position (P_{n ⊂ 1..k}) that corresponds to the actual position in the vasculature. In so doing, an even more accurate position may be determined. This may be more accurate than known techniques in which a spatial encoder at the entry point of the catheter is used to determine the said position. Said known techniques are subject to axial variations in vasculature path length and consequently suffer from a positioning error due to the malleable nature of the vasculature. In so doing, system SY facilitates easier re-visitation of previously-measured positions in the vasculature.

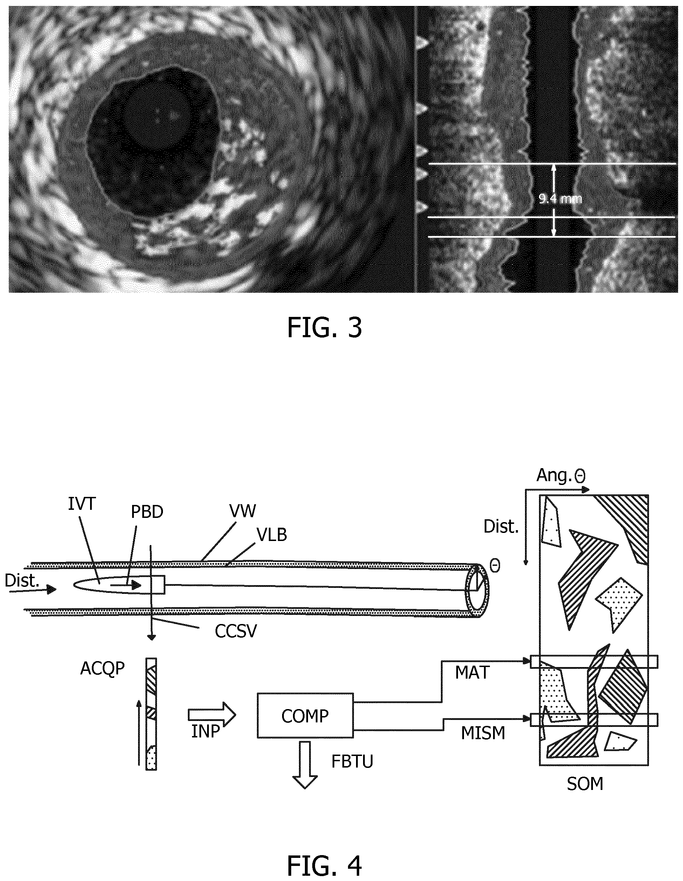

Subsequently, optional display unit DISP in Fig. 1 may be used to indicate the result of the comparison. Display unit DISP may for example display i) at least a portion of the reference map, i.e. SOM in exemplary Fig. 4, including a representation of the reference measurement data at the determined position P_{n ⊂ 1..k} that corresponds to the actual position in the vasculature VA; and ii) a representation of the actual measurement data at the actual position in the vasculature VA. The representations of the data may for example be IVUS/ OCT images, or features of said images as described in more detail below.

In one implementation, system SY further includes optional classification unit CU. Classification unit CU classifies the actual measurement data and the reference measurement data with a feature set. A statistical learning algorithm may optionally be used in the classification. The statistical learning algorithm may be trained in a supervised or unsupervised manner. Alternatively a predetermined algorithm may be used for the classification. In this implementation the comparator unit COMP is configured to determine the position P_{n⊂ 1..k} that corresponds to the actual position in the vasculature by comparing the feature set of the actual measurement data with the feature set of the reference measurement data. The feature set of the actual measurement data and the feature set of the reference measurement data may each include at least one element such as an intensity distribution of the respective data in a radial direction R and/ or a rotational direction θ with respect to an axis A - A' defined by a lumen LU of the vasculature VA, a representation of tissue type in a radial direction R and/ or a rotational direction θ with respect to an axis A - A' defined by a lumen LU of the vasculature VA, a representation of plaque type in a radial direction R and/ or a rotational direction θ with respect to an axis A - A' defined by a lumen LU of the vasculature VA, an average intensity of the respective data, an entropy of the respective data, a geometrical parameter of the vasculature VA such as an average diameter of the vasculature VA or a cross-sectional area of the vasculature VA or a plaque thickness or a vessel wall thickness. In general the use of feature set elements that correspond to data at a higher level of generality than raw IVUS/ OCT imaging data allows a simpler and/ or a faster comparison to be made. The use of feature set elements that are rotationally-invariant, such as the average diameter of the vasculature VA or a cross-sectional area of the vasculature VA, further simplifies the comparison since it is not necessary to compare the feature set elements for multiple rotational angles θ.

When the feature set includes at least one of the elements: the representation of tissue type in a radial direction R and/ or a rotational direction θ with respect to an axis A - A' defined by a lumen LU of the vasculature VA, the representation of plaque type in a radial direction R and/ or a rotational direction θ with respect to an axis A - A' defined by a lumen LU of the vasculature VA; the comparator unit COMP may be further configured to determine the position P_{n ⊂ 1..k} that corresponds to the actual position in the vasculature by comparing at least one of i) a dominant tissue type and/ or plaque, ii) a closest tissue type and/ or plaque to the lumen LU, iii) a closest tissue type and/ or plaque to the vessel wall VW, iv) a tissue type and/ or plaque having the largest extent in the radial direction R and/ or in the rotational direction θ, of the feature set elements. The use of these feature set elements at this level of generality may provide for a faster comparison by comparator COMP.

In another implementation the actual measurement data and the reference measurement data are both measured in a radial direction R with respect to an axis A - A' defined by a lumen LU of the vasculature VA and correspond to a region RE of the vasculature VA bordered by the lumen LU and a vessel wall VW. In this implementation the comparator unit COMP may be configured to determine the position P_{n ⊂ 1..k} that corresponds to the actual position in the vasculature by comparing the feature set of the actual measurement data with the feature set of the reference measurement data within the region RE. Optionally, only the data within region RE may be used in the comparison. By excluding data from outside region RE, which might correspond to anomalous features, a faster and more reliable comparison may be provided.

In another implementation the actual measurement data and the reference measurement data are both measured in a radial direction R with respect to the axis A - A' defined by the lumen LU of the vasculature VA and correspond to a region RE of the vasculature bordered by the lumen LU and a vessel wall VW. In this implementation the actual measurement data and the reference measurement data corresponding to the bordered region RE are both scaled in the radial direction R to a common radial range such that the comparator unit determines the position P_{n ⊂ 1..k} that corresponds to the actual position in the vasculature by comparing the actual measurement data and the reference measurement data at corresponding radii within the common radial range. This scaling may improve the reliability of the position determination because deformations in the vasculature, e.g. deformations during the cardiac phase, may be compensated-for.

In another implementation system SY includes optional image registration unit IRU and optional angiogram-providing unit APU illustrated in Fig. 1. Angiogram-providing unit APU is configured to provide an angiogram based on coronary computed tomography angiogram data corresponding to the vasculature VA. Image registration unit IRU is configured to register the angiogram with the reference map, i.e. SOM in exemplary Fig. 4. In so doing the actual position may be determined in the angiogram. Improved position determination may be provided by the ability to track the actual position in the angiogram since the angiogram, being generated using X-ray radiation, has a different sensitivity to imaged features than IVUS/ OCT. Moreover, a user's familiarity with an angiogram may further facilitate improved position determination by the user.

In another implementation the reference measurement data has a first angular sampling interval rotationally θ around an axis A - A' defined by a lumen LU of the vasculature VA, and a first axial sampling interval along the axis A - A' defined by the lumen LU of the vasculature VA, and the actual measurement data has a second angular sampling interval rotationally θ around the axis A - A' defined by the lumen LU of the vasculature VA, and a second axial sampling interval along the axis A - A' defined by the lumen LU of the vasculature VA. In this implementation the first angular sampling interval and/ or the first axial sampling interval is less than the second angular sampling interval and/ or the second axial sampling interval, respectively. This allows for the use of "sparse" actual measurement data when re-visiting the vasculature and may provide a faster determination of the actual position with respect to the reference map.

In another implementation the reference measurement data has a first penetration depth in a radial direction R with respect to an axis A - A' defined by a lumen LU of the vasculature VA, and the actual measurement data has a second penetration depth in the radial direction R. The penetration depth may be adjusted by for example changing a power or a frequency of IVUS/ OCT probe signals. In this implementation the second penetration depth is less than the first penetration depth. This likewise allows for the use of "sparse" actual measurement data in a radial direction when re-visiting the vasculature and may provide a faster determination of the actual position with respect to the reference map.

In another implementation system SY further includes optional coronary plaque burden computation unit CPBCU. Coronary plaque burden computation unit CPBCU is configured to compute a coronary plaque burden metric based on the actual measurement data or the reference measurement data. Thereto coronary plaque burden computation unit CPBCU receives data from data-providing unit DPU and/ or map-providing unit MPU, and may be configured to compute one or more coronary plaque burden algorithms such as that discussed in document "Comparison between MDCT and Grayscale IVUS in a Quantitative Analysis of Coronary Lumen in Segments with or without Atherosclerotic Plaques"; by Falcao, J. L. A. A, et. al.; Arq. Bras. Cardiol. 2015 April; 104(4):315-23.

Any of the modules: map providing unit MPU, data-providing unit DPU, comparator COMP, classification unit CU, coronary plaque burden computation unit CPBCU, angiogram-providing unit APU, image registration unit IRU, described in relation to Fig. 1 may be provided by hardware modules or software modules, or a combination of hardware and software modules, which carry out the described functions to achieve the described effects. Moreover the various implementations or aspects may be combined in order to achieve other beneficial effects.

Fig. 2 illustrates a flowchart corresponding to a method MET of determining a position of an OCT or IVUS catheter CA in a vasculature VA. Method MET includes the steps of:
providing PRM a reference map SOM including reference measurement data at each of a plurality of positions P_{1.k} of the OCT or IVUS catheter CA in the vasculature VA;
providing actual measurement data PAMD from an actual position in the vasculature;
determining DET from the plurality of positions P_{1.k} of the OCT or IVUS catheter in the vasculature VA, a position P_{n ⊂ 1..k} that corresponds to the actual position in the vasculature, based on a comparison of the actual measurement data and the reference measurement data; wherein the reference measurement data and the actual measurement data are either i) OCT data or ii) IVUS data.

Method MET may be used by system SY in Fig. 1, and therefore any aspect or implementation disclosed in relation to corresponding system SY may also be implemented in method MET.

In accordance with one implementation, method MET comprises the aforementioned method steps and these may be recorded as instructions which when executed on a computer cause the computer to carry out the method steps.

The invention thus tackles one or more of the above-mentioned drawbacks by providing a means to support navigation with recordings from intravascular imaging. These can be typical cross-sectional IVUS or OCT recordings as clinically used, or in a second option "lighter" recordings with maybe reduced angular sampling and/or imaged penetration depth. The latter may serve as a quicker option of recording information that can be applied in the background of the intervention, i.e. when no explicit recording is triggered by the user, e.g. during catheter insertion. The goal in this additional option would be to only record as much information as necessary for pattern generation to not impede the clinical workflow.

Using the recordings, lumen and wall border can be delineated which provide detail about the local vessel geometry. As this geometry is subject to deformation (e.g. across the cardiac phase) only rough measures of it (such as average diameter or cross-sectional area) can be used to get vague location specific features.

In addition, the depth information of the intravascular images may contain information about the space between lumen and outer wall border. This depth information can be grouped into similar classes. With reference to Fig. 3, which illustrates pattern generation based on tissue typing using virtual histology, these may be anatomically well-defined classes such as tissue types [1], or just re-appearing characteristics of yet un-defined anatomical meaning. With further reference to Fig. 3, while the transducer is pulled through a vessel segment, cross-sectional images of the vessel are generated. In those, lumen and vessel wall are delineated and tissue types are classified in the area between both borders using the technique disclosed in citation [1]. The spatial distribution of plaque types as well as some rough aspects of the vessel geometry, e.g. average diameter, thickness of plaque, can be used to store orientation maps of unique patterns along the vessel. Therefore, each location defined by a position along (e.g. the centerline of) the vessel and an angular offset, can be characterized by such a class or a multi-dimensional code describing e.g. partial association to several groups or different sources of information at different imaging depths. At each point along the vessel the recorded cross-section can therefore be converted into an angular pattern of classes that can be seen as a unique characterization of this location where the acquired information has been stripped to an amount necessary for serving the purpose.

After visiting several places along the vessel centerline, an orientation map as shown in Fig. 4 can be generated, which stores the distribution of patterns along the vessel. The patterns in the orientation map can be interpreted as encodings of the imaging information per location. If certain locations should be revisited, comparisons between the currently recorded pattern and the stored orientation map can identify matches to earlier recordings and provide orientation as well as a means for navigation within the vessel. The encoding of semantic information as patterns of a discrete set of classes compresses the data by discarding irrelevant information and noise and enables robust comparisons by applying simpler similarity scores.

The distance coordinate of the map can be quantitatively justified by e.g. using a spatial encoder together with the catheter or a fixed pullback speed or, alternatively just represents the order of patterns along the vessel without exactly knowing the spacing between them. This distinction is important for cases where no automatic pullback is performed with the transducer, but a manual positioning is carried out with a changing movement speed of the catheter tip.

Note that the lateral coordinate, i.e. pointing from centerline outwards, may be distorted by vessel and soft tissue deformation during the cardiac cycle and the intervention. A standard orientation map may therefore reduce the vessel to a tunnel of unit radius with patterns on its walls. Extensions of this invention may make use of this coordinate e.g. by describing each point on the tunnel wall by a vector each element of which links to a feature/class index at a certain distance bin to the centerline. Along these lines, a vector could further capture differently relevant aspects across the lateral image direction such as the most dominant feature, the feature closest to lumen, features closest to wall, biggest connected feature etc.

Fig. 4: illustrates an exemplary IVUS imaging embodiment of the invention that uses pullback through a vessel while imaging the vessel. With reference to Fig. 4, IVUS transducer IVT is pulled back along pullback direction PBD along which a distance Dist is measured. Vessel wall VW and vessel lumen border VLB are imaged. In each cross-section, i.e. acquired pattern ACQP, the vessel wall VW and lumen are delineated and the area between them is classified into one of several classes; i.e. the hatched/ dotted regions in the Stored Orientation Map SOM. In this example each location is represented by a scalar only, which is projected onto a unit circle around the current location on the vessel centerline. In a general case this could be a multidimensional characteristic. The matching unit can read from, and possibly also write to, an orientation map, aforementioned characteristic as a function of distance and angle, of already visited places along the centerline. Matching unit COMP in Fig. 4 receives acquired pattern ACQP as input INP and compares the pattern with patterns in stored orientation map SOM. If a match MAT, is found, this indicates the actual position in the stored orientation map SOM, i.e. the reference map. A mismatch is indicated at position MISM. Feedback to a user FBTU may then be provided. Thus, by comparing the current pattern with patterns in the database, the matching unit can decide whether the location was visited before, notify the user, who possibly tagged a destination location, or provide information previously logged for this location.

### Main components:

*Intravascular imaging device.* An imaging device, IVUS, OCT or others, may be inserted into a vessel using a catheter. At each location along the vessel centerline, the device records cross-sectional depth-images as illustrated in Fig. 3. Viewing these 2D images in polar coordinates may provide concatenated vectors of depth information for each angular sample.

The invention may alternatively incorporate other intravascular imaging methods which provide information about the surrounding vessel area along the centerline. These include but are not restricted to near infrared spectroscopy, i.e. NIRS, (fluorescence) angioscopy or thermoscopy [2]. Translating imaging information into an orientation map would also provide mapping across modalities, in case they share the information necessary to predict common classes from the set. This potentially even includes coronary computed tomography angiogram, i.e. CCTA, data, where the centerline could be labelled with information that can be similarly found during the intervention with intravascular imaging, e.g. certain coronary plaque types.

*Pattern generation unit.* A pattern generation unit may classify each depth information vector at each angular position into one of several classes from a discrete set. In the general case this encoding step does not necessarily translate the depth information into a scalar, but can also describe the location by a vector. Across the entire angular range for a given location, this yields a characteristic pattern of encodings.

*Orientation Map.* For each visited location these encoding patterns can be stored in an orientation map. The map provides a pattern distribution along the vessel, see Fig. 4. A user may link certain informative tags to each of the patterns, e.g. "stenosis", "landmark 1" etc.). The location of a new pattern with respect to other patterns along the "distance coordinate", see Fig. 4, can be defined using a spatial encoder at the entry point of the catheter, a well-defined pullback speed, or just in terms of order, i.e. "next", "previous", in case only the moving direction, but not the speed is known.

*Matching Unit.* A matching unit compares the current pattern with patterns in the orientation map using some similarity score. It can therefore (1) provide orientation within the context the probe has already seen by identifying similar and dissimilar patterns, (2) guide the user when trying to revisit certain past locations, possibly tagged with information, or (3) notify the user when a location has been already visited. The matching unit provides a direct interface to the user to accept requests or provide feedback.

A key component of the invention may be the exploitation of "interpreted" imaging data to augment the otherwise unreliable spatial geometry of the vessel. In the simplest case, spatial information may be discarded completely, matching being performed solely based on the class patterns.

The encoding of imaging information can be achieved by algorithms as in [1] or be learned in a data-driven way. The latter can be done in a (1) supervised, or (2) unsupervised manner. In the first case the developer pre-defines a set of desired classes such as tissue types and labels the imaging data with it. Using supervised statistical learning, a function is learnt that maps imaging information into the set of these classes. In the second case, no annotations, but only imaging data is available. In this case unsupervised learning can learn the data distribution and cluster it into a set of reappearing patterns, i.e. modes, into which new information is classified. Those do not necessarily have any strict anatomical meaning.

The learned information can be supported by features of the spatial geometry. Although the geometry, but also the recorded pattern information, may be subject to some changes over time, the richness of encoded information may still ensure a robust identification and distinction via similarity score.

In general, the similarity score can measure similarity between matrices or vectors such as a Euclidean distance. The score may be adapted to handle the rotational degree of freedom, i.e. account for the fact that the probe might be rotated around its longitudinal axis when revisiting a location. The score therefore may detect similarity of the current pattern with any possible rotation of the stored pattern in the orientation map, i.e. any offset shift in the angular direction. Comparisons in the spectral domain could account for this comparison efficiently.

In use, a user may first navigate through the vessel where unknown places are filled into the orientation map. An illustration of the orientation map may be provided to the user such as a colored axis with a marker indicating the current position. The user is able to tag each location with descriptors or information if desired. In case the user revisits already known locations he may be notified by the orientation map and its marker. New locations, which may lie between already known locations, are simply filled into the orientation map. In this way associations between multiple recordings of the same site can be achieved.

In another use case, the user aims to re-visit a previously tagged landmark. The system may notify him once a place of similar encoding pattern is reached. This also provides a means of making sure a location is in fact the desired location.

The invention may be used in applications that involve IVUS or intravascular OCT imaging such as plaque burden or lesion monitoring for acute coronary syndromes, i.e. ACS, or stable angina. Another field of application is in stenting and follow-up imaging. It is applicable to both coronary and peripheral vessels. In these applications the invention can facilitate navigation with the catheter, re-identify previously visited locations to validate correspondences, aggregate matched data and reduce angiography imaging during the intervention.

The intervention may require less angiography scans, and this has a benefit for the patient and clinician. Moreover, navigation with the described approach may alleviate the need for a spatial encoder or other overview images as from a computed tomography angiogram, i.e. CTA.

Any of the method steps disclosed herein may be recorded in the form of instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

In summary, system for determining a position of an OCT or IVUS catheter in a vasculature, has been described. The system includes a map-providing unit, a data-providing unit, and a comparator unit. The map-providing unit is configured to provide a reference map including reference measurement data at each of a plurality of positions of the OCT or IVUS catheter in the vasculature. The data-providing unit is configured to provide actual measurement data from an actual position in the vasculature. The comparator unit is configured to determine from the plurality of positions of the OCT or IVUS catheter in the vasculature, a position that corresponds to the actual position in the vasculature, based on a comparison of the actual measurement data and the reference measurement data. The reference measurement data and the actual measurement data are either i) OCT data or ii) IVUS data.

Various implementations, aspects, embodiments and options have been described in relation to system SY, and it is noted that the various embodiments may be combined to achieve further advantageous effects.

## Claims

1. System (SY) for determining a position of an OCT or IVUS catheter (CA) in a vasculature (VA); the system (SY) comprising:
a map-providing unit (MPU) configured to provide a reference map (SOM) including reference measurement data at each of a plurality of positions (P_{1.k}) of the OCT or IVUS catheter (CA) in the vasculature (VA);
a data-providing unit (DPU) configured to provide actual measurement data from an actual position in the vasculature (VA);
a comparator unit (COMP) configured to determine from the plurality of positions (P_{1.k}) of the OCT or IVUS catheter in the vasculature, a position (P_{n ⊂ 1..k}) that corresponds to the actual position in the vasculature, based on a comparison of the actual measurement data and the reference measurement data; and
wherein the reference measurement data and the actual measurement data are either i) OCT data or ii) IVUS data.

2. The system (SY) according to claim 1 further comprising a classification unit (CU);
wherein the classification unit (CU) is configured to classify the actual measurement data and the reference measurement data, respectively at the actual position in the vasculature (VA) and at the plurality of positions (P_{1.k}) of the OCT or IVUS catheter (CA) in the vasculature (VA), with a feature set, optionally using a statistical learning algorithm;
and wherein the comparator unit (COMP) is configured to determine the position (P_{n ⊂ 1..k}) that corresponds to the actual position in the vasculature by comparing the feature set of the actual measurement data with the feature set of the reference measurement data.

3. The system (SY) according to claim 2 wherein the feature set includes at least one of the following elements: an intensity distribution of the respective data in a radial direction (R) and/ or a rotational direction (θ) with respect to an axis (A - A') defined by a lumen (LU) of the vasculature (VA), a representation of tissue type in a radial direction (R) and/ or a rotational direction (θ) with respect to an axis (A - A') defined by a lumen (LU) of the vasculature (VA), a representation of plaque type in a radial direction (R) and/ or a rotational direction (θ) with respect to an axis (A - A') defined by a lumen (LU) of the vasculature (VA), an average intensity of the respective data, an entropy of the respective data, a geometrical parameter of the vasculature (VA) such as an average diameter of the vasculature (VA) or a cross-sectional area of the vasculature (VA) or a plaque thickness or a vessel wall thickness.

4. The system (SY) according to claim 3 wherein the feature set includes at least one of the elements: the representation of tissue type in a radial direction (R) and/ or a rotational direction (θ) with respect to an axis (A - A') defined by a lumen (LU) of the vasculature (VA), the representation of plaque type in a radial direction (R) and/ or a rotational direction (θ) with respect to an axis (A - A') defined by a lumen (LU) of the vasculature (VA); and
wherein the comparator unit (COMP) is configured to determine the position (P_{n ⊂ 1..k}) that corresponds to the actual position in the vasculature by comparing at least one of i) a dominant tissue type and/ or plaque, ii) a closest tissue type and/ or plaque to the lumen (LU), iii) a closest tissue type and/ or plaque to the vessel wall (VW), iv) a tissue type and/ or plaque having the largest extent in the radial direction (R) and/ or in the rotational direction (θ), of the feature set elements.

5. The system (SY) according to claim 1 wherein the actual measurement data and the reference measurement data are both measured in a radial direction (R) with respect to an axis (A - A') defined by a lumen (LU) of the vasculature (VA) and correspond to a region (RE) of the vasculature (VA) bordered by the lumen (LU) and a vessel wall (VW);
wherein the comparator unit (COMP) is configured to determine the position (P_{n ⊂ 1..k}) that corresponds to the actual position in the vasculature by comparing the feature set of the actual measurement data with the feature set of the reference measurement data within the region (RE).

6. The system (SY) according to any previous claim wherein the actual measurement data and the reference measurement data each correspond to detected OCT or IVUS signals that are measured rotationally (θ) around an axis (A - A') defined by a lumen (LU) of the vasculature (VA), or wherein the actual measurement data corresponds to detected OCT or IVUS signals that are measured rotationally (θ) around an axis (A - A') defined by a lumen (LU) of the vasculature (VA) and the reference measurement data is simulated OCT or IVUS data indicative of OCT or IVUS signals measured rotationally (θ) around the axis (A - A') and which simulated data is derived from MRI image data corresponding to the vasculature (VA).

7. The system (SY) according to claim 6 wherein the actual measurement data and the reference measurement data are both measured in a radial direction (R) with respect to the axis (A - A') defined by the lumen (LU) of the vasculature (VA) and correspond to a region (RE) of the vasculature bordered by the lumen (LU) and a vessel wall (VW); and
wherein the actual measurement data and the reference measurement data corresponding to the bordered region (RE) are both scaled in the radial direction (R) to a common radial range such that the comparator unit determines the position (P_{n ⊂ 1..k}) that corresponds to the actual position in the vasculature by comparing the actual measurement data and the reference measurement data at corresponding radii within the common radial range.

8. The system (SY) according to claim 1 further comprising an image registration unit (IRU) and an angiogram-providing unit (APU);
wherein the angiogram-providing unit (APU) is configured to provide an angiogram based on coronary computed tomography angiogram data corresponding to the vasculature (VA); and
wherein the image registration unit (IRU) is configured to register the angiogram with the reference map (SOM).

9. The system (SY) according to claim 1 wherein the reference measurement data has a first angular sampling interval rotationally (θ) around an axis (A - A') defined by a lumen (LU) of the vasculature (VA), and a first axial sampling interval along the axis (A - A') defined by the lumen (LU) of the vasculature (VA); and wherein the actual measurement data has a second angular sampling interval rotationally (θ) around the axis (A - A') defined by the lumen (LU) of the vasculature (VA), and a second axial sampling interval along the axis (A - A') defined by the lumen (LU) of the vasculature (VA); and wherein the first angular sampling interval and/ or the first axial sampling interval is less than the second angular sampling interval and/ or the second axial sampling interval, respectively.

10. The system (SY) according to claim 1 wherein the reference measurement data has a first penetration depth in a radial direction (R) with respect to an axis (A - A') defined by a lumen (LU) of the vasculature (VA);
wherein the actual measurement data has a second penetration depth in the radial direction (R); and
wherein the second penetration depth is less than first penetration depth.

11. The system (SY) according to claim 1 further comprising a coronary plaque burden computation unit (CPBCU) configured to compute a coronary plaque burden metric based on the actual measurement data or the reference measurement data.

12. The system (SY) according to claim 1 further comprising a display unit (DISP); wherein the display unit (DISP) is configured to display i) at least a portion of the reference map (SOM) including a representation of the reference measurement data at the determined position (P_{n ⊂ 1..k}) that corresponds to the actual position in the vasculature (VA); and ii) a representation of the actual measurement data at the actual position in the vasculature (VA).

13. The system (SY) according to claim 1 further comprising an OCT or IVUS catheter (CA) configured to provide the reference measurement data to the map-providing unit (MPU) and/ or the actual measurement data to the data-providing unit (DPU).

14. Method (MET) of determining a position of an OCT or IVUS catheter (CA) in a vasculature (VA); the method (MET) comprising:
providing (PRM) a reference map (SOM) including reference measurement data at each of a plurality of positions (P_{1.k}) of the OCT or IVUS catheter (CA) in the vasculature (VA);
providing actual measurement data (PAMD) from an actual position in the vasculature;
determining (DET) from the plurality of positions (P_{1.k}) of the OCT or IVUS catheter in the vasculature (VA), a position (P_{n ⊂ 1..k}) that corresponds to the actual position in the vasculature, based on a comparison of the actual measurement data and the reference measurement data;
wherein the reference measurement data and the actual measurement data are either i) OCT data or ii) IVUS data.

15. Computer program product comprising instructions which when executed on a computer cause the computer to carry out the method of claim 14.
